# EUROPÄISCHE PATENTANMELDUNG

(11) **EP 3 539 597 A1**
(43) Veröffentlichungstag der Anmeldung: **18.09.2019**
(21) Anmeldenummer: 19160708.4
(22) Anmeldetag: 05.03.2019
(51) Int. Cl.: A61M 5/315, A61F 9/00, A61K 9/00, A61M 5/32

(54) **APPLIKATOR**

(30) Priorität: 16.03.2018 DE 102018106217
(71) Anmelder: AMW GmbH, 83627 Warngau (DE)
(72) Erfinder: Partenhauser, Alexandra, 83059 Kolbermoor (DE); Wiedenbauer, Magnus, 83714 Miesbach (DE)
(74) Vertreter: Beckord & Niedlich Patentanwälte PartG mbB

(57) **Zusammenfassung**

Die vorliegende Erfindung betrifft einen Applikator (1, 1'), insbesondere zur intravitrealen Applikation, umfassend einen Spritzenkörper (10) mit einem in einem Gehäuseteil (11a) beweglich gelagerten Kolben (20, 27, 29) und eine Kanüle (2), wobei zumindest ein Abschnitt der Kanüle (50) und/oder des Spritzenkörpers (10) gekrümmt ausgebildet sind.

## Beschreibung

Die vorliegende Erfindung betrifft einen Applikator, insbesondere zur intravitrealen Applikation eines Arzneimittels bzw. zur Verwendung in der Augenheilkunde, umfassend einen Spritzenkörper mit einem in einem Gehäuseteil beweglich gelagerten Kolben und einer Kanüle.

Die Darreichung von Wirkstoffen in oder an das Auge ist begrenzt für Wirkstoffe mit einer kurzen Halbwertszeit, da deren Wirksamkeit im intraoccularen Gewebe begrenzt ist. Daher ist die wirksamste Art der Darreichung eines Wirkstoffs auch an das Hintersegment (Posterior Segment) des Auges die direkte Verabreichung in den Glaskörper ("Corpus Vitrearum" umgangssprachlich auch kurz "Vitreus" genannt) (Maurice, D.M. (1983), Ocular Inflammation Ther. 1: 97-102; Lee, V.H.L. et al. (1989), in: Retina. T.E. Ogden and A.P. Schachat, Eds., St. Louis: CV Mosby, Vol. 1, S. 483-98; und Olsen, T.W. et al. (1995), Invest. Ophthalmol. Vis. Sci. 36: 1893-1903).

Die hierbei eingesetzte Technologie der intravitrealen Injektion, also eine Injektion des Arzneimittels in den Glaskörper, hat zwar vielversprechende Resultate gezeigt. Ungünstigerweise wird eine solche intravitreale Applikation jedoch von den Patienten meist als sehr unangenehm empfunden. Dies liegt in erster Linie daran, dass der Behandler sich mit dem Applikator, also beispielsweise einer herkömmlichen Spritze, üblicherweise im Visusbereich bzw. Sichtbereich, oft sogar direkt in der Visuslinie, des Patienten bewegen muss, um mit der Kanüle an einer definierten Stelle in den Glaskörper einzudringen. Bei der Annäherung der Kanüle an das Auge wird insbesondere der im Sichtbereich liegende Spritzenkörper mit der in einer Flucht liegenden Spitze der Kanüle stark vergrößert wahrgenommen.

Ungünstigerweise muss zudem bei einer großen Anzahl an Arzneimitteln, wie beispielsweise Glucocorticoiden, aufgrund der kurzen intravitrealen Halbwertszeit die intravitreale Injektion in kurzen Abständen wiederholt werden, um den Wirkstoffspiegel aufrecht zu erhalten. Dieser sich in kurzen Zeitabständen wiederholende Prozess erhöht die Wahrscheinlichkeit von unerwünschten Nebenwirkungen, wie beispielsweise einer Netzhautablösung, Endophthalmitis und Grauem Star (Maurice, D.M. (1983), Ocular Inflammation Ther. 1: 97-102; Olsen, T.W. et al. (1995), Invest. Ophthalmol. Vis. Sci. 36: 1893-1903; Kwak, H.W. und D'Amico, D. J. (1992), Arch. Ophthalmol. 110: 259-66).

Um bei einer Behandlung die Häufigkeit einer intravitrealen Injektion zu reduzieren, kann eine Verabreichung eines bioabbaubaren Implantats unter die Lederhaut des Auges (Sclera) oder in den subconjunktivalen oder suprachoroidalen Raum erfolgen, wie beispielsweise in US 4,863,457, WO 95/13765, WO 00/37056, oder in Could et al., Can. J. Ophthalmol. 29(4):168-171 (1994) und in Apel et al., Curr. Eye Res. 14:659-667 (1995) beschrieben. Hierdurch kann zwar die Häufigkeit der für den Patienten unangenehmen Injektionen reduziert werden und auch die Gefahr der Nebenwirkungen reduziert werden, jedoch besteht nach wie vor das Problem, dass die Injektion als solche als unangenehm empfunden wird. Hinzu kommt, dass die Applikatoren, die zur Applikation eines Implantats geeignet sind, in den meisten Fällen nicht so klein dimensioniert werden können wie bei anderen, beispielsweise flüssigen Arzneimitteln, was bei der Applikation den unangenehme Eindruck auf den Patienten weiter erhöhen kann.

Es ist eine Aufgabe der vorliegenden Erfindung, einen Applikator der eingangs genannten Art anzugeben, welcher besser zur Verabreichung von Arzneimitteln an besonders sensiblen oder schwer zugänglichen Stellen geeignet ist, wie insbesondere dem Auge, um das oben beschriebene Problem der unangenehmen Wahrnehmung des Patienten im Zuge einer intravitrealen Applikation zu reduzieren oder ganz zu vermeiden und so die Akzeptanz des Applikators zu verbessern.

Diese Aufgabe wird durch eine Applikator gemäß Patentanspruch 1 gelöst.

Wie bereits eingangs beschrieben, umfasst dieser Applikator einen Spritzenkörper mit einem in einem, vorzugsweise im Wesentlichen zylindrisch ausgebildeten, Gehäuseteil beweglich (bzw. verschieblich) gelagerten Kolben. Dieses Gehäuseteil kann insbesondere als ein Rohrabschnitt ausgebildet sein, in dessen Hohlraum der Kolben bewegt werden kann. Der Kolben kann, wie bei einer üblichen Spritze, auch einen äußeren Betätigungsteil umfassen, an dem der Kolben bewegt wird, also beispielsweise eine Kolbenstange mit einer am hinteren Ende verbreiterten Andruckfläche oder dergleichen für den Daumen des Behandlers.

Weiterhin umfasst der Applikator eine am vorderen Ende des Spritzenkörpers angeordnete Kanüle. Diese Kanüle kann, wie später noch erläutert wird, lösbar am Spritzenkörper angeordnet oder fest mit diesem verbunden sein. Es wird an dieser Stelle darauf hingewiesen, dass sich die Begriffe "vorne" und "hinten" im Rahmen der vorliegenden Patentanmeldung auf die übliche Orientierung bei der Nutzung eines solchen Applikators beziehen, d. h. hinten ist die Seite, an der sich der Kolben befindet, auf den z. B. der Behandler drückt, um den Kolben im Spritzenkörper zu verschieben, und vorne ist die Spitze der Kanüle, mit der die Kanüle zur Verabreichung des Arzneimittels in das Körperteil des Patienten eindringen kann bzw. bezogen auf den Spritzenkörper die Seite, an der die Kanüle am Spritzenkörper angeschlossen ist oder wird.

Erfindungsgemäß ist zumindest ein Abschnitt der Kanüle und/oder des Spritzenkörpers gekrümmt ausgebildet. Mit anderen Worten, der Applikator ist so ausgebildet, dass die Gesamtlinie vom hinteren Ende, d. h. dem Kolben, des Applikators zum distalen, d. h. dem vorderen Ende der Kanüle, zumindest in dem vorderen Bereich, d. h. im Bereich der Kanüle oder dem der Kanüle benachbarten vorderen Bereich des Spritzenkörpers, von einer geraden Linie abweicht, vorzugsweise bogenförmig ist.

Dadurch, dass bei dem erfindungsgemäßen Applikator der relativ große Spritzenkörper mit dem Kolben nicht mehr in einer Linie mit der Spitze der Kanüle liegt, kann mit dem Applikator ein Arzneimittel in den Glaskörper eingebracht werden, ohne dass dabei der größte Teil des Applikators, nämlich der Spritzenkörper mit dem Kolben in der Visuslinie, bzw. nahe der Visuslinie des Patienten liegt. Bei der Anwendung ist der Applikator daher für den Patienten kaum sichtbar bzw. liegt allenfalls im Randbereich des Visus und somit ist die Applikation für den Patienten erheblich angenehmer.

Mit dem erfindungsgemäßen Applikator können, wie später noch erläutert wird, Arzneimitteln in beliebigen Darreichungsformen, insbesondere auch Implantate, in den Glaskörper eingebracht werden. Auch wenn der Applikator besonders gut für eine Anwendung in der Augenheilkunde, insbesondere zur intravitrealen Applikation geeignet ist, ist er jedoch nicht auf eine solche Verwendung beschränkt, sondern kann auch für andere Zwecke zum Einbringen von Arzneimitteln in den Körper eines Menschen oder eines Tieres genutzt werden, an denen beispielsweise die Zugänglichkeit der Spritze entlang einer geraden Linie nicht so geeignet ist.

Weitere, besonders vorteilhafte Ausgestaltungen und Weiterbildungen der Erfindung ergeben sich aus den abhängigen Ansprüchen sowie aus der nachfolgenden Beschreibung, wobei die unabhängigen Ansprüche einer Anspruchskategorie auch analog zu den abhängigen Ansprüchen und Ausführungsbeispielen bzw. Beschreibungsteilen einer anderen Anspruchskategorie weitergebildet sein können und insbesondere auch einzelne Merkmale verschiedener Ausführungsbeispiele bzw. Varianten zu neuen Ausführungsbeispielen bzw. Varianten kombiniert werden können.

Wie erwähnt, kann sich die Krümmung lediglich auf die Kanüle beziehen, was in den meisten Fällen einfacher und daher bevorzugt ist. Prinzipiell schließt das aber nicht aus, dass die Krümmung auch im Spritzenkörper realisiert wird, also beispielsweise in dem zur Kanüle benachbarten vorderen Gehäuseteil des Spritzenkörpers. Grundsätzlich wäre es auch möglich, dass die Krümmung auf die Kanüle und den Spritzenkörper verteilt ist, d. h., dass sowohl ein Bereich des Spritzenkörpers gekrümmt ist und diese Krümmung in der Kanüle fortgesetzt wird.

Vorzugsweise verläuft die Krümmung in dem gekrümmten Abschnitt im Wesentlichen gleichmäßig, d. h. in einem Bogen (im Rahmen von üblichen Toleranzen).

Die Krümmung über die gesamte Länge des Applikators kann z. B. durch einen Winkel definiert werden, der die Abweichung einer am distalen Ende der Kanüle, d. h. an der Kanülenöffnung, anliegenden Tangente, von einer Längsachse des Kolbens angibt. Dabei kann die Längsachse des Kolbens so definiert werden, dass sie z. B. mit der Verschieberichtung des Kolbens im hinteren Gehäuseteil übereinstimmt. Die Tangente verläuft dagegen in Längsrichtung der Kanüle an ihrem distalen Ende bzw. führt das distale Ende in einer auslaufenden geraden Richtung weiter. Besonders bevorzugt beträgt der so definierte Winkel mindestens 5 °, vorzugsweise mindestens 10 °. Weiterhin ist bevorzugt, dass der Winkel zumindest 30 °, vorzugsweise maximal 90° beträgt, d. h. dass sich die Krümmung maximal über einen Viertelkreis erstreckt.

Welche Krümmung ideal ist, kann von verschiedenen Bedingungen abhängen, beispielsweise vom Applikationsort, also ob beispielsweise der Applikator zur intravitrealen Applikation dient und wenn ja, an welcher Stelle bevorzugt die Kanüle in den Glaskörper eindringen soll, zum anderen aber auch von der Menge und/oder der Darreichungsform des einzubringenden Arzneimittels, also beispielsweise im Falle eines Implantats dessen Größe und Ausgestaltung.

Bei einer im Wesentlichen gleichmäßigen Krümmung beträgt der Krümmungsradius vorzugsweise mindestens 0.1 cm. Besonders bevorzugt beträgt der Krümmungsradius mindestens 1 cm, bevorzugt mindestens 3 cm. Der Krümmungsradius beträgt vorzugsweise maximal 20 cm, besonders bevorzugt maximal 10 cm.

Vorzugsweise weist die Kanüle einen über deren Länge gleichbleibenden Durchmesser auf, besonders bevorzugt einen gleichbleibenden Innendurchmesser. Dies ist insbesondere dann vorteilhaft, wenn mit dem Applikator ein Implantat injiziert werden soll, wie dies später noch erläutert wird.

Der Innendurchmesser der Kanüle beträgt vorzugsweise mindestens 0.5 mm, ganz besonders bevorzugt mindestens 0.8 mm. Um andererseits die Einstichstelle möglichst gering zu halten, beträgt der Außendurchmesser der Kanüle vorzugsweise maximal 2,5 mm, besonders bevorzugt maximal 2 mm.

Die Kanüle sollte vorzugsweise an ihrem distalen Ende spitz zulaufen, um einerseits leicht in das betroffene Körperteil, insbesondere den Glaskörper, eindringen zu können und andererseits die Beschädigung des Glaskörper dabei so gering wie möglich zu halten. Ganz besonders bevorzugt weist die Kanüle einen so genannten "Facettenschliff" auf, d. h. sie ist schräg angeschliffen und an deren vorderen Spitze relativ scharf, wobei der zurückliegende Teil der Spitze, also die Hälfte der Wandung der Kanüle, welche sich am zurückliegenden Teil des distalen Endes der Kanüle befindet, ist dazu relativ stumpf ausgebildet, sodass die Kanüle beim Einstechen kein Loch einstanzt, sondern lediglich einen kleinen Schnitt an der scharfen Seite verursacht.

Auch die Länge der Kanüle kann, in Abhängigkeit vom konkreten Applikationszweck, gewählt sein. Bevorzugt beträgt die Nettolänge der Kanüle mindestens 1 cm. Vorzugsweise beträgt die Nettolänge maximal 10 Zentimeter. Unter der Nettolänge wird hierbei die Länge der Kanüle ohne den Teil der Kanüle verstanden, der sich bei ordnungsgemäß montiertem Zustand innerhalb des Spritzenkörpers befindet.

Es sei an dieser Stelle darauf hingewiesen, dass der Applikator in einer bevorzugten Variante auch eine ansteckbare bzw. abnehmbare Kanüle aufweisen kann. Dabei ist es auch möglich, dass die Kanüle und der Spritzenkörper so ausgebildet sind, dass die Kanüle erst kurz vor der Anwendung am Spritzenkörper montiert wird, aber ein späteres Lösen nach der Anwendung dann nicht mehr möglich ist und der Applikator mit montierten Kanüle entsorgt bzw. einem Recycling zugeführt werden muss. Bei einer festen Anbringung der Kanüle im Applikator ist es eine besonders einfache Montagemöglichkeit, das zum Spritzenkörper weisende Ende der Kanüle in eine entsprechende vordere Öffnung des Spritzenkörpers einzustecken und dort zu verkleben oder mechanisch zu verankern.

Grundsätzlich kann ein Applikator auch ohne Arzneimittel ausgeliefert werden, sodass ein Behandler bzw. Anwender vor Ort zunächst das Arzneimittel in den Applikator einbringt, um es im Anschluss dem Patienten zu verabreichen. Besonders bevorzugt enthält der Applikator aber bereits das Arzneimittel.

Beispielsweise könnte, wie später noch genauer erläutert wird, ein Arzneimittel in einer Darreichungsform als Implantat mit geeigneten Abmessungen direkt in der Kanüle angeordnet sein, beispielsweise in Form eines länglichen Stäbchens mit einem Außendurchmesser, der geringer als der Innendurchmesser der Kanüle ist.

Bei einer besonders bevorzugten Variante weist der Applikator eine Depoteinheit, umfassend das Arzneimittel, auf. Alternativ kann der Applikator auch mit einer Depoteinheit, in welcher sich das Arzneimittel befindet, erst vom Anwender bestückt werden, d. h. der Applikator und die zugehörige Depoteinheit werden getrennt angeliefert und erst unmittelbar vor der Anwendung miteinander gekoppelt, bzw. die Depoteinheit im Applikator, in der Regel im Spritzenkörper, untergebracht. Insbesondere auch diesen Fällen kann es bevorzugt sein, wenn auch die Kanüle ebenfalls vom Spritzenkörper getrennt geliefert wird und erst vor der Anwendung mit diesem verbunden wird, gegebenenfalls auch mit der Depoteinheit gekoppelt wird, die dann wiederum mit dem Spritzenkörper gekoppelt, bzw. in diesen eingeschoben, wird.

Unter einer "Depoteinheit" ist vorliegend eine Aufnahmeeinheit zu verstehen, welche das Arzneimittel aufnimmt und z. B. in einer definierten Position, insbesondere im Spritzenkörper, halten kann. Beispielsweise kann die Depoteinheit eine Halterung für ein Implantat umfassen, beispielsweise mit einer Art Federvorrichtung, um das Implantat an einer bestimmten Stelle im Spritzenkörper zu halten. Dabei ist die Halterung so ausgebildet, dass sie das Implantat sicher hält, aber nachgibt, wenn das Implantat durch Betätigung des Kolbens, beispielsweise wie dies noch erläutert wird mit einem am Kolben befestigten Mandrin, durch die Kanüle herausgeschoben wird. Ebenso kann es sich bei der Depoteinheit aber auch um ein Reservoir für ein Arzneimittel in Form einer flüssigen oder halbfesten Formulierung, wie z. b. eines Gels, einer Paste, oder einer vergleichbaren Darreichungsform handeln.

Mit einer solchen Depoteinheit ist es auch möglich, ein Mehr-Komponenten-Arzneimittel (z. B. ein Zwei-Komponenten-Arzneimittel) erst vor Ort bzw. direkt vor der Anwendung zur Bildung der finalen Darreichungsform zusammenzubringen, indem beispielsweise die Depoteinheit ein Mehrkammersystem, insbesondere 2-Kammersystem, aufweist. Bei einer Betätigung des Kolbens, kann zunächst eine Komponente des Arzneimittels aus einer ersten Kammer in eine weitere Kammer, in welcher sich eine weitere Komponente des Arzneimittels befindet, verbracht werden u.s.w., bevor dann schließlich die Mischung durch eine weitere Betätigung des Kolbens insgesamt als fertig zubereitetes Arzneimittel verabreicht wird, d. h. durch die Kanüle in das Körperteil, insbesondere den Glaskörper, eingebracht wird.

Die Depoteinheit kann wie erwähnt bevorzugt im Spritzenkörper angeordnet sein, z. B. direkt vor der Kanüle, sie kann aber auch in der Kanüle angeordnet sein oder beispielsweise zwischen Kanüle und Spritzenkörper montiert sein. In diesem Fall kann die Depoteinheit auch als zusätzlicher, vorderer Teil des Spritzenkörpers angesehen werden. Insbesondere kann dieser Teil dann auch gegebenenfalls gekrümmt ausgebildet sein.

Die Depoteinheit kann, wie ebenfalls erwähnt, fest im Applikator verbaut aber auch ankoppelbar bzw. auswechselbar sein, je nachdem, was im konkreten Anwendungsfall bzw. bei dem jeweiligen Arzneimittel gewünscht ist.

Wird das Arzneimittel direkt in der Kanüle, beispielsweise im vorbeschriebenen Fall das Implantat, eingelegt bzw. gelagert, kann auf eine solche Depoteinheit verzichtet werden.

Um das Arzneimittel durch bzw. aus der Kanüle herauszubewegen, weist der Applikator vorzugsweise einen mit dem beweglichen Kolben gekoppelten Mandrin auf. Unter einem solchen Mandrin wird ein Führungsstab verstanden, um das Arzneimittel beispielsweise vom Spritzenkörper und/oder aus einer Depoteinheit in die Kanüle hineinzuführen, gegebenenfalls durch die Kanüle hindurchzuführen und/oder aus der Kanüle herauszudrücken. Vorzugsweise ist der Mandrin zumindest abschnittsweise flexibel ausgebildet, um eine Bewegung des Mandrins durch einen gekrümmten Bereich der Kanüle oder des vorderen Spritzenteils zu erlauben. Flexibel heißt hierbei, dass sich der Mandrin in die Kanüle hineinbiegt und anpassen kann, d. h. das Material des Mandrins sollte bevorzugt verformbar, insbesondere elastisch verformbar, sein.

Insbesondere wenn der Mandrin durch einen Teil der Kanüle verlaufen soll, ist dieser relativ dünn ausgebildet und weist einen Außendurchmesser auf, welcher an den Innendurchmesser der Kanüle angepasst ist, d. h. vorzugsweise lediglich geringfügig kleiner als der Innendurchmesser der Kanüle ist. Im Spritzenkörper, insbesondere in einer Depoteinheit für das Arzneimittel, sofern eine solche Depoteinheit verwendet wird, kann sich bevorzugt eine Führung für den Mandrin befinden.

Der Mandrin kann an einem vorderen Ende des Kolbens angebracht sein. Je nach Ausgestaltung und Materialwahl kann der Mandrin gegebenenfalls auch integrativ mit dem Kolben beispielsweise in einem Spritzgussverfahren hergestellt sein.

Insbesondere sofern der Applikator bereits bei der Lieferung an den Anwender ein Arzneimittel umfasst, können die Kanüle und/oder der Spritzenkörper und/oder die Depoteinheit ein Verschlusselement aufweisen, um das Arzneimittel beispielsweise vor einem vorzeitigen Kontakt mit der äußeren Umgebung zu schützen und/oder um zu verhindern, dass das Arzneimittel vorzeitigt nach vorne aus dem Applikator herausgelangt. Unter einem solchen Verschlusselement kann also jeglicher Verschluss bzw. Verschlussvorrichtung zur entsprechenden Sicherung des Arzneimittels verstanden werden, weshalb das Verschlusselement auch als "Sicherheitsverschluss" bezeichnet werden könnte. Vorzugweise kann es sich hierbei um eine Art Lamelle oder eine Folienwand oder dergleichen im Spritzenkörper handeln. Ein weiteres bevorzugtes Beispiel wäre ein Sicherheitsverschluss mit einen Drehverschluss oder Ähnliches.

Dieser Sicherheitsverschluss kann vorzugsweise automatisch bei Auslösen des Applikators geöffnet werden, wie z. B. ein Verschlusselement in Form einer Folie, bevorzugt mit einer Sollbruchstelle, die bei Betätigen des Kolbens aufgebrochen wird. Das Verschlusselement ist bevorzugt aus flexiblen Material, wie beispielsweise eine Folie, sodass es sich nach den Aufbrechen z. B. seitlich flexibel wegbewegen kann und das Durchtreten des Arzneimittels ermöglicht.

Besonders bevorzugt kann hierzu der Applikator, insbesondere am Kolben oder am Mandrin, ein Öffnungselement, wie zum Beispiel eine kleine Spitze aufweisen, um damit das Verschlusselement beim Betätigen des Kolbens zwangsläufig zu öffnen bzw. zu durchstoßen.

Ebenso kann der Sicherheitsverschluss auch so ausgebildet sein, dass er zunächst in einer vom Auslösen des Applikators unabhängigen Aktion vom Anwender manuell (von außen) entfernt werden kann, z. B. durch Abziehen oder Herausziehen einer Folie, eines kleinen Schiebers oder Stopfens.

Besonders bevorzugt handelt es sich hier um ein spritzenkörper- bzw. kanüleninternes Verschlusselement, welches für eine Positionierung des Arzneimittels in einem Abstand vom distalen Ende des Spritzenkörpers bzw. der Kanüle sorgt. Mit anderen Worten, es handelt sich also beispielsweise nicht nur um ein einfache Kappe, die außen aufgesteckt wird, sondern zumindest um eine Art Verschlussstopfen oder dergleichen, mit einem Abschnitt der am distalen Ende in das Lumen des Kanüle und/oder des Spritzenkörpers bzw. der Depoteinheit hineinragt und sich somit im geschlossenen Zustand innerhalb der Kanüle bzw. des Spritzenkörpers oder des Depots befindet und sicher verhindert, dass das Arzneimittel bis nach vorne zur Öffnung rutschen oder fließen kann.

Der Spritzenkörper weist bevorzugt zumindest eine Griffhilfe auf, an welcher ein Anwender beispielsweise mit zwei Fingern den Spritzenkörper ergreifen kann, während er mit dem Daumen auf den Kolben drückt. Ein Beispiel für solche Griffhilfen sind Griffmulden an gegenüberliegenden Seiten der äußeren Mantelfläche, des im Wesentlichen zylindrischen kolbenseitigen Gehäusesteils des Spritzenkörpers.

Vorzugsweise kann der Spritzenkörper auch eine, vorzugsweise lösbar am Gehäuse montierte bzw. montierbare, Abstützhilfe aufweisen, welche beispielsweise zum Abstützen an einem Körperteil eines Patienten genutzt werden kann, um den Applikator bei der Applikation möglichst ruhig zu halten. Diese Abstützhilfe kann beispielsweise je nach Anwendungsfall so ausgebildet sein, dass der Applikator am Kopf, z. B. an der Stirn oder am Kinn, abgestützt werden kann.

Der Applikator wird bevorzugt mit einer geeigneten Umverpackung ausgestattet und ausgeliefert. Diese Umverpackung weist bevorzugt einen formstabilen Aufnahmeraum für den Spritzenkörper und/oder die Kanüle auf. Der Aufnahmeraum kann beispielsweise in einer Art Basis bzw. einem erstem Gehäuseteil der Umverpackung angeordnet sein, welche dann mit einem Deckel (als zweites Gehäuseteil) abgedeckt wird, vorzugsweise einer Folie, beispielsweise einer Blisterfolie, überzogen wird. Besonders bevorzugt ist ein solcher Aufnahmeraum zumindest bereichsweise an eine Kontur des Applikator angepasst, damit der Applikator fest im Aufnahmeraum gehalten wird und sich darin nicht während des Transports bewegt. Bei Nutzung eines ausreichend formstabilen Aufnahmeraumes kann auf einen Spitzenschutz, also beispielsweise eine Endkappe, auf der Kanüle verzichtet werden, da ja keine Gefahr besteht, dass es vor dem Auspacken zu einem unbeabsichtigten Kontakt mit der scharfen Spitze der Kanüle kommt, beispielsweise beim Transport oder der Lagerung.

Eine besonders kostengünstige Möglichkeit zur Herstellung eines geeigneten ersten Gehäuseteils mit einem Aufnahmeraum wäre ein Tiefziehen einer geeigneten Folie, wobei im Tiefziehprozess der Aufnahmeraum als eine Art Aufnahmemulde in die Folie eingebracht wird. Hierbei sollte bevorzugt darauf geachtet werden, dass die Folie im geformten Zustand eine ausreichende Stabilität aufweist, d. h. wie ein Hartkunststoff wirkt. Nach Einlegen des Spritzenkörpers mit der Kanüle braucht dieser dann wie erwähnt z. B. nur noch mit Blisterfolie überzogen werden.

Die Materialien für die verschiedenen Komponenten des Applikators können in Abhängigkeit vom genauem Aufbau und Anwendungszweck des Applikators gewählt werden.

So können beispielsweise der Spritzenkörper und/oder der Kolben vorzugsweise aus Kunststoff, wie beispielsweise PET und/oder Polycarbonat und/oder Polypropylen, hergestellt werden.

Vorzugsweise ist dafür gesorgt, dass alle Komponenten bzw. Teile, die einen Kontakt mit dem Arzneimittel haben, aus arzneimittelkompatiblen Materialien hergestellt werden, wie beispielsweise Edelstahl, Styrole oder Polycarbonate
Insbesondere die Kanüle ist vorzugsweise möglichst formstabil bzw. starr ausgebildet, sodass sie sich während der bestimmungsgemäßen Verwendung des Applikators nicht verformt. Dabei kann es von dem verwendeten Material und den Abmessungen abhängen, mit welchen Toleranzen die Kanüle starr ist. Ein bevorzugtes Material für die Kanüle ist medizinischer Edelstahl.

Das Arzneimittel, welches mit Hilfe des erfindungsgemäßen Applikators injiziert wird, kann, wie bereits erwähnt, in flüssiger und/oder halbfester und/oder fester Form vorliegen, wie beispielsweise in der Monograpie für Zubereitungen zur Anwendung am Auge beschrieben (Ph. Eur 9.4). Dabei können flüssige Formen beispielsweise als wässrige oder ölige Lösung, Emulsion oder Suspension eines oder mehrerer Wirkstoffe vorgesehen sein. Halbfeste Zubereitungen sind beispielsweise Gele; feste Zubereitungen sind beispielsweise Implantate, in welche ein oder mehrere Wirkstoffe in eine Matrix eingebettet ist bzw. sind. Solche Zubereitungen sind bevorzugt steril, wobei eine Sterilisierung durch eine antimikrobielle Konservierung und/oder durch eine Bestrahlung, beispielsweise mit UV-Licht, erfolgen kann.

Besonders bevorzugt liegt das Arzneimittel in Form von Liposomen, Mikropartikeln, als Suspension, als Lösung, Gel und/oder Implantat, insbesondere liegt das Arzneimittel in Form eines Implantats, vor.

Prinzipiell kann ein Wirkstoff, welcher sich in dem Arzneimittel befindet, aus unterschiedlichen Klassen ausgewählt sein.

Bevorzugt umfasst das Arzneimittel einen Wirkstoff mit Wirkung auf den Glucokortikoid-Rezeptor, wie beispielsweise Dexamethason, Prednisolon oder Hydrocortison.

Alternativ oder zusätzlich kann das Arzneimittel bevorzugt einen Wirkstoff umfassen, welcher ausgewählt ist aus VEGF-Inhibitoren, wie beispielsweise Ranibizumab und/oder Aflibercept, und/oder einen Wirkstoff aus der Gruppe der Antiinfektiva, wie beispielsweise Aciclorvir, Chloramphenicol, Fluorochinolonacetonid und/oder Gentamicin.

Besonders bevorzugt ist ein Wirkstoff, welcher sich in dem Arzneimittel befindet, ausgewählt aus Dexamethason, Methotrexat, Vincristin oder Hydrocortison, insbesondere aus Dexamethason.

Wie bereits eingangs erläutert, eignet sich der erfindungsgemäße Applikator vorteilhaft für alle Stellen des menschlichen und tierischen Körpers, welche schwer zugänglich sind bzw. für welche der Applikator eine vorteilhafte Anwendung ermöglicht. Vorzugsweise wird der Applikator in der Augenheilkunde, bevorzugt zur Therapie von krankhaften Veränderungen des Auges, insbesondere zur Behandlung der altersbedingten Makuladegeneration, Retinopathie, wie beispielsweise der diabetischen Retinopathie oder der Retinopathia pigmentosa, zur Behandlung des Grauen Stars (Cataract), des Grünen Stars, der Hornhautentzündung (Keratitis), Color Vision Defect, Glaucoma, Herpes simplex-Infection, Endophthalmitis, Chorioditis, Iritis, Makulaödem, bevorzugt des diabetischen Makulaödems (DMÖ) oder des Makulaödems als Folge eines retinalen Venenverschlusses, Uveitis, Entzündungen des posterioren Augensegments, Cytomegalie-Virus-Retinitis, Neovascularisation, bakterielle und virale Infektionen, Augentumorerkrankungen und/oder zur Immunsuppresion und/oder -stimulation eingesetzt. Insbesondere wird der Applikator zur Behandlung des Makulaödems und von Entzündungen des posterioren Augensegments eingesetzt.

Die Erfindung wird im Folgenden unter Hinweis auf die beigefügten Figuren anhand von Ausführungsbeispielen noch einmal näher erläutert. Dabei sind in den verschiedenen Figuren gleiche Komponenten mit identischen Bezugsziffern versehen. Es zeigen schematisch:
Figur 1 eine seitliche Ansicht eines ersten Ausführungsbeispiels eines erfindungsgemäßen Applikators, hier mit einer Depoteinheit für ein Arzneimittel in Form eines Implantats (Depoteinheit und Kanüle jeweils im Schnitt dargestellt);
Figur 2 eine seitliche Ansicht des vorderen Teils eines zweiten Ausführungsbeispiels eines erfindungsgemäßen Applikators, hier mit einem Arzneimittel in Form eines Implantats in der Kanüle (Kanüle im Schnitt);
Figur 3 eine seitliche Ansicht des vorderen Teils eines dritten Ausführungsbeispiels eines erfindungsgemäßen Applikators, hier mit einer Depoteinheit für ein Arzneimittel in Form einer Lösung (Depoteinheit und Kanüle jeweils im Schnitt dargestellt);
Figur 4 eine seitliche Ansicht des vorderen Teils eines vierten Ausführungsbeispiels eines erfindungsgemäßen Applikators, hier mit einer Depoteinheit mit einem Zwei-Komponenten-Arzneimittel (Depoteinheit und Kanüle jeweils im Schnitt dargestellt) in einem Ausgangszustand;
Figur 5 eine seitliche Ansicht des vorderen Teils des Applikators gemäß Figur 4, jedoch hier in einem Präparationszustand nach Mischung der beiden Komponenten und vor Montage der Kanüle;
Figur 6 eine seitliche Ansicht des vorderen Teils des Applikators gemäß den Figuren 4 und 5, jedoch hier in einem anwendungsbereiten Zustand nach Montage der Kanüle;
Figur 7 eine seitliche Draufsicht auf ein Ausführungsbeispiel eines erfindungsgemäßen Applikators mit einer Umverpackung;
Figur 8 ein Ausführungsbeispiel eines erfindungsgemäßen Applikators bei einer intravitrealen Applikation eines Arzneimittels.

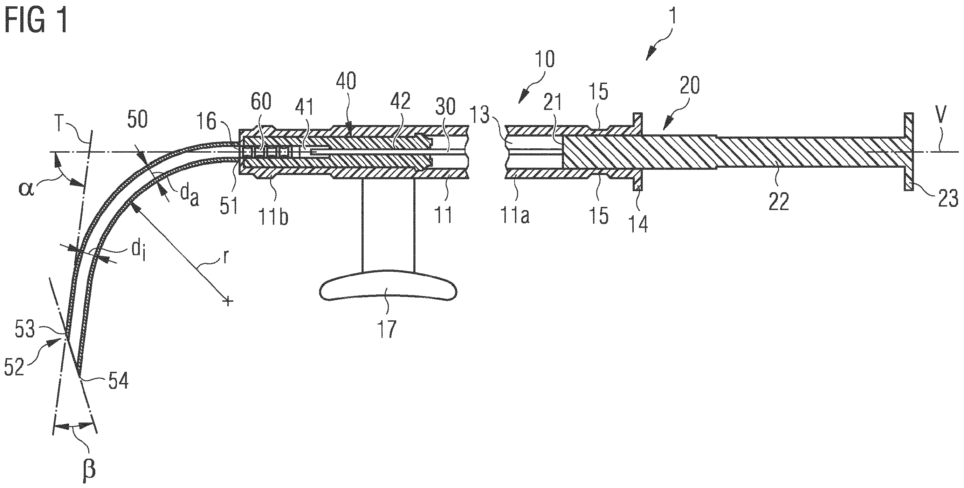

Bei dem in Figur 1 dargestellten bevorzugten Ausführungsbeispiel eines erfindungsgemäßen Applikators 1, handelt es sich um einen Applikator 1, um ein Arzneimittel 60 in der Darreichungsform eines Implantats 60 in den Glaskörper eines Patienten einzubringen.

Der Applikator 1 weist hier einen Spritzenkörper 10 mit einem Wesentlichen zylinderförmigen Gehäuse 11 auf. An einer vorderen Stirnseite des Gehäuses 11 ist hier (zentral) eine Kanüle 50 aus Edelstahl montiert. In einem von der Kanüle 50 weg weisenden hinteren Gehäuseteil 11a befindet sich ein Kolben 20, der in einem Hohlraum 13 bzw. Kolbenraum 13 des Gehäuses 11 entlang einer Längsachse V des Kolbens 20 bzw. des Spritzenkörpers 10 verschiebbar im Innenraum des betreffenden Gehäuseteils 11a gelagert ist. Dieser Kolben 20 weist eine nach hinten aus dem Gehäuse 11 herausragende Kolbenstange 22 auf, an der sich endseitig eine Andruckplatte 23 befindet, auf die ein Anwender, beispielsweise mit dem Daumen, von hinten drücken kann, um den Kolben 20 weiter in das Gehäuse 11 des Spritzenkörpers 10 hineinzuschieben.

In einem zur Kanüle 50 weisenden vorderen Gehäuseteil 11b befindet sich eine Depoteinheit 40, welche ebenfalls im Wesentlichen zylindrisch aufgebaut ist und welche einen Außendurchmesser aufweist, der an den Innendurchmesser des Innenraums des vorderen Gehäuseteils 11b des Spritzenkörpers 10 angepasst ist. Diese Depoteinheit 40 ist beispielsweise einfach von vorne in eine stirnseitige Öffnung 16 des Gehäuses 11 eingeschoben und durch Vorsprünge an der Außenseite bzw. Mantelfläche der Depoteinheit 40 und entsprechende Gegenvorsprünge im Inneren des vorderen Gehäuseteils 11b des Spritzenkörpers 10 im Gehäuse 11 verrastet, so dass die Depoteinheit 40 nach dem Einschieben nicht wieder herausgezogen werden kann. Das Gehäuse 11 mit dem hinteren Gehäuseteil 11a und dem vorderen Gehäuseteil 11b ist einteilig aus Kunststoff hergestellt, z. B. im Spritzgussverfahren

An der zur Kanüle 50 weisenden Stirnseite befindet sich in der Depoteinheit 40 ein Hohlraum 41, in welchem hier ein stäbchenförmiges Implantat 60 angeordnet ist, das radiale Einkerbungen aufweist, damit das Implantat 60 flexibler ist und sich gut durch eine gebogene Kanüle hindurchschieben lässt. Alternativ könnte das Implantat 60 auch aus mehreren Teilstücken bestehen und/oder aus einem besonders flexiblen Stoff gebildet sein. Am stirnseitigen freien Ende des Hohlraums 41 befindet sich zentral (also mittig in der Stirnseite) eine etwas erweiterte Endbohrung 16, in der die Kanüle 50 mit ihrem spritzenkörperseitigen bzw. zur Depoteinheit 40 weisenden Ende verankert, beispielsweise eingeklebt, ist. Der Durchmesser der erweiterten Endbohrung 16 ist mindestens so groß, wie der Außendurchmesser dₐ der Kanüle 50 und der Innendurchmesser des Hohlraums 41 ist maximal so groß, wie der Innendurchmesser dᵢ der Kanüle 50, so dass das Implantat 60 ohne blockiert zu werden vom Hohlraum 41 aus in die Kanüle 50 eingeschoben werden kann.

Vom Hohlraum 41 aus erstreckt sich außerdem eine Führungsbohrung 42 längs durch die Depoteinheit 40 nach hinten in Richtung des Kolbens 20.

Der Kolben 20 weist an seinem zur Kanüle 50 bzw. zur Depoteinheit 40 weisenden Stirnseite 21 einen Mandrin 30 auf, d. h. dieser Mandrin 30 ist mit dem stirnseitigen Ende des Kolbens 20 gekoppelt. Der Mandrin 30 ist im vorliegenden Fall ein biegsames bzw. flexibles, zylinderförmiges, sehr langes Stäbchen, welches durch die Führungsbohrung 42 der Depoteinheit 40 in deren Hohlraum 41 hineinragt und welches dazu dient, bei einer Betätigung des Kolbens 20 durch Verschieben in das Gehäuse 11 des Spritzenkörpers 10 hinein das Implantat 60 in die Kanüle 50 und durch diese hindurch in den Glaskörper des Patienten zu befördern. Hierzu muss der Mandrin 30 die richtige Länge aufweisen und entsprechend muss auch der Kolben 20 lang genug sein, damit der Verschiebeweg in das Gehäuse 11 des Spritzenkörpers hinein ausreicht, dass der Mandrin 30 bis an das distale Ende 52 der Kanüle 50 heranreicht, um das Implantat 60 vollständig in den Glaskörper zu befördern. Um die wesentlichen Objekte etwas kompakter in Figur 1 darstellen zu können, sind hier der Spritzenkörper 10 bzw. das Gehäuse 11 und der Mandrin 30 in einem mittleren Abschnitt geschnitten worden, wodurch symbolisiert werden soll, dass sowohl das Gehäuse 11 des Spritzenkörpers 10, als auch der Mandrin 30 entsprechend länger sind. Im vorliegenden Fall muss beispielsweise die Länge der Kolbenstange 22 an die Länge der Kanüle 50 einschließlich der Länge des Implantats 60 angepasst sein.

Diese Kanüle 50 ist hier gekrümmt, wobei der Krümmungsradius r 5 cm beträgt. Die Krümmung beträgt etwas weniger als einen Viertelkreis, d. h. der Winkel α der Abweichung einer Tangente T, welcher an dem distalen Ende 52 der Kanüle 50 angelegt ist, von der Richtung der Längsachse V bzw. der Bewegungsrichtung des Kolbens 20, beträgt hier ca. 83°.

Die Kanüle selber weist hier über die gesamte gleichmäßige Krümmung einen konstanten Innendurchmesser von 1.3 mm auf, sodass ein Implantat 60 mit einem Durchmesser von 1.2 mm gut durch die Kanüle geführt werden kann. Das Implantat 60 selber ist hier so ausgebildet, dass es aus mehreren Teilstücken besteht, die durch die Krümmung der Kanüle 50 gut hindurchgeschoben werden können. Alternativ oder zusätzlich kann das Implantat flexibel ausgebildet sein. Entsprechend ist, wie erwähnt, der Mandrin 30 flexibel ausgebildet.

Vorzugsweise ist auch der Außendurchmesser da der Kanüle 50 über die gesamte Länge der Kanüle 50 konstant, woraus folgt, dass auch die Dicke der Wandung der Kanüle 50 konstant ist. Der Außendurchmesser da beträgt hier bevorzugt 1.5 mm.

An ihrem distalen Ende 52 weist die Kanüle eine Spitze 54 auf, indem das distale Ende 52 der Kanüle 50 schräg in einem sogenannten "Facettenschliff" angeschliffen wurde, wobei der Winkel β zwischen der schrägen Fläche 53 und der Tangente T approximativ 15° beträgt. Die schräge Fläche 53 ist nicht geradlinig, sondern - wie bei einem Facettenschliff üblich abgeknickt. Durch diesen Facettenschliff ist nur die spitze Seite bzw. in etwa die Hälfte der Wandung der Kanüle scharf und der zurückliegende Teil der Wandung, also ungefähr der halbe Umfang der Kanülenwandung, stumpf. Dadurch wird nur ein feiner Schnitt an der Spitze im Glaskörper erzeugt und kein Loch ausgestanzt, wenn die Kanüle 50 in den Glaskörper eindringt.

Die Nettolänge der Kanüle 50 beträgt hier in etwa 8 cm, wobei unter Nettolänge die freie Länge zu verstehen ist, ab der Stelle, an der die Kanüle 50 aus der Endbohrung 16 am Hohlraum 41 der Depoteinheit 40 herausragt, bis zum distalen Ende 52 am vordersten Punkt der Spitze 54.

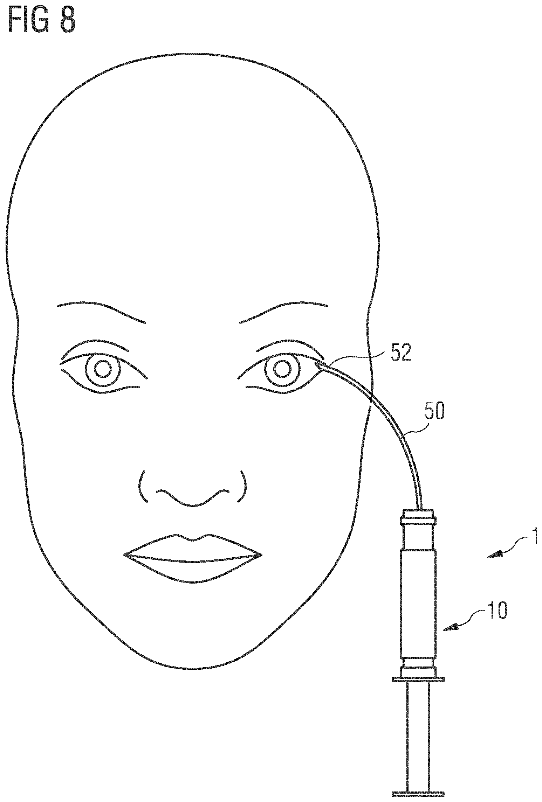

Eine mögliche Verwendung dieses Applikators 1 zum Einbringen eines Implantats 60 in den Glaskörper eines Patienten ist in Figur 8 schematisch dargestellt. Wie hier zu sehen ist, ist es aufgrund der Krümmung der Kanüle 50 möglich, dass der Behandler, beispielsweise ein Augenarzt, den Applikator 1 seitlich vom Auge des Patienten außerhalb dessen Gesichtsfelds oder allenfalls im Randbereich des Gesichtsfelds an das Auge heranführt, sodass der Patient den Applikator erst relativ spät und nur außen im Gesichtsfeld sieht und nicht in der Visusachse, wie dies bei einem herkömmlichen Applikator der Fall ist.

Zur besseren Handhabung befinden sich an der äußeren Mantelfläche des Gehäuseteils 11a nahe dem kolbenseitigen Ende jeweils auf gegenüberliegenden Seiten Griffmulden 15 und direkt am Ende ein Kragen 14. Kragen 14 und Griffmulde 15 bilden zusammen eine Art Griffstück, welches der Behandler gut mit zwei Fingern greifen kann, während er gleichzeitig mit dem Daumen hinten auf die Andruckplatte 23 am Ende der Kolbenstange 22 drückt.

Als weitere Bedienungshilfe kann der Applikator 1 eine Abstützhilfe 17 aufweisen, welche hier symbolisch dargestellt ist. Diese kann auch am Gehäuse 11 außen beweglich montiert bzw. montierbar sein. Mit dieser Abstützhilfe kann der Behandler beispielsweise den Applikator am Kinn des Patienten abstützen, um so einen ruhigen Bezugspunkt zum Kopf des Patienten zu haben.

Wie erwähnt, ist die Ausführungsform gemäß Figur 1 nur ein Beispiel für möglich Varianten zur Unterbringung des Arzneimittels im Applikator. So ist es beispielsweise nicht zwingend erforderlich, dass sich das Arzneimittel in einer Depoteinheit befindet.

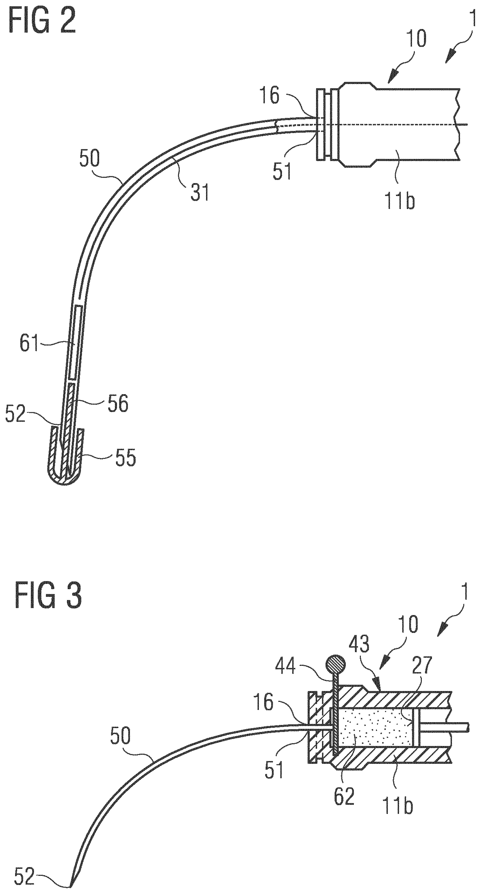

Hierfür zeigt Figur 2 ein Beispiel. In Figur 2 ist, wie auch in den weiteren Figuren 3 bis 6 jeweils nur der vordere Gehäuseteil 11 des Spritzenkörpers 10 dargestellt, da das kolbenseitige hintere Gehäuseteil 11a und der Kolben 20 in ähnlicher oder identischer Weise ausgebildet sein kann, wie bei dem Ausführungsbeispiel gemäß Figur 1.

Wie in Figur 2 dargestellt ist, befindet sich hier das Arzneimittel 61 in Form eines stäbchenförmigen Implantats 61 bereits in einer definierten Position im vorderen Bereich der Kanüle 50. Die Kanüle 50 ist daher hier so ausgebildet, dass sie in ihrem vorderen Bereich wieder ein Stück geradlinig verläuft. Auch der Mandrin 31 ist so lang, dass er bereits ein ganzes Stück in die Kanüle 50 hineinragt, bis unmittelbar an oder kurz vor das Implantat 61. Um das Implantat 61 in dieser Position zu halten und auch vor Umgebungseinflüssen zu schützen, befindet sich vorne auf der Kanüle eine Spritzenkappe 55 mit einem Stopfen 56 als spritzenkörper- bzw. kanüleninternes Verschlusselement 56, der in das Lumen der Kanüle 50 hineinragt und das Implantat 60 in Position hält.

Die Kanüle 50 ist hier mit ihrem spritzenkörperseitigen Ende 51 einfach in eine zentrale Bohrung des Gehäuses 11 des Spritzenkörpers 10 eingeklebt bzw. auf sonstige Weise befestigt.

Zur Anwendung muss dann vom Behandler nur noch die Spitzenkappe 55 abgezogen werden.

Figur 3 zeigt ein weiteres Ausführungsbeispiel, mit dem ein Arzneimittel 62 in halbfester Form, zum Beispiel als Gel oder als Paste, appliziert werden kann. Das Arzneimittel 62 befindet sich hier wieder in einer Depoteinheit 43, welche einen Hohlraum aufweist. Der Kolben weist hier beispielsweise eine in den Hohlraum hineinragende Verlängerung mit einer endseitigen Kolbenfläche 27 auf, welche sich über den gesamten Querschnitt des Hohlraums der Depoteinheit 43 erstreckt, sodass bei einem Einschieben des Kolbens die Kolbenfläche 27 den Hohlraum verkleinert und das halbfeste Arzneimittel 62 in die Kanüle 50 und weiter am distalen Ende aus der Kanüle 50 herausgedrückt wird. Es sei an dieser Stelle darauf hingewiesen, dass die Menge des Arzneimittels 62 groß genug sein muss, dass sie nicht nur die Kanüle 50 auffüllt, sondern auch noch genügend Arzneimittel in den Glaskörper befördert wird. Dementsprechend muss das Volumen des Hohlraums der Depoteinheit 43 passend zur Kanüle 50 berechnet werden und entsprechend mit Arzneimittel 62 befüllt werden. Bei der schematischen Darstellung in Figur 3 ist dies nicht berücksichtigt.

Um einen vorzeitigen Kontakt des Arzneimittel 62 mit der Umgebung bzw. Luftkontakt zu verhindern, befindet sich zwischen dem zum Spritzenkörper 10 weisenden Ende der Kanüle 50 und dem Hohlraum der Depoteinheit 53 ein Verschlusselement 44 in Form einer Folie bzw. Lamelle, welche an einem nach außen hinausragenden Zugelement (welches hier nur durch einen Punkt dargestellt ist, der ein rundes Griffstück symbolisieren soll) seitlich herausgezogen werden kann. Alternativ könnte diese Lamelle als sehr dünne Folie mit einer Sollbruchstelle oder dergleichen in der Mitte ausgebildet sein, welche zerreißt, wenn das Arzneimittel 62 durch den Kolben nach vorne gedrückt wird.

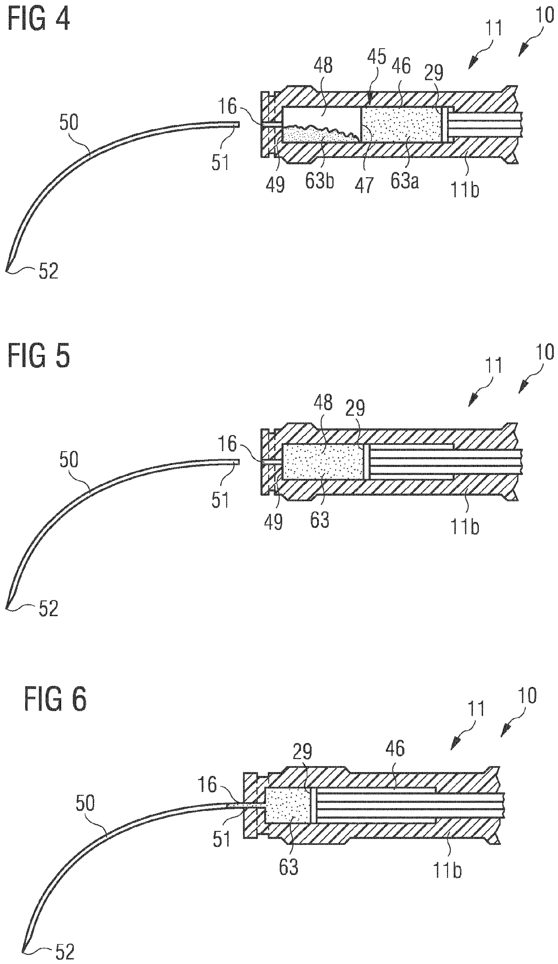

Anhand der Figuren 4 bis 6 wird nun eine weitere Variante mit einer Depoteinheit 45 erläutert, welche geeignet ist, Komponenten 63a und 63b eines Zwei-Komponenten-Arzneimittels 63 getrennt voneinander zu halten und erst unmittelbar vor oder bei der Anwendung unter Bildung des Arzneimittels 63 zusammenzuführen.

Hierzu weist die Depoteinheit 45 zwei durch eine dünne, folienartige Trennwand 47 getrennte zylindrische Kammern 46, 48 auf. In der zum Kolben weisenden hinteren Kammer 46 befindet sich beispielsweise eine Medikamentenkomponente 63a in flüssiger Form. Hierbei kann es sich beispielsweise um Wasser für Injektionszwecke mit verschiedenen Zusätzen (wie beispielsweise Puffersalze, Tenside und anderen pharmazeutisch akzeptablen Zusatzstoffen) oder um eine ölige Lösung umfassend mittelkettige Triglyceride handeln. In einer zur Kanüle 60 weisenden vorderen Kammer 48 befindet sich eine zweite granulat-artige Arzneimittelkomponente 63b, im vorliegenden Fall in Form von Mikro- und/oder Nanopartikeln.. Diese vordere Kammer 48 ist durch eine weitere dünne Folie 49 als Verschlussmittel 49 von einer zentralen Öffnung 16 getrennt, in welche das zum Spritzenkörper weisenden Ende der Kanüle eingesetzt werden kann. In die erste, hintere Kammer 46 ragt wieder eine Kolbenverlängerung mit einer endseitigen Kolbenfläche 29 hinein, wobei die Kolbenfläche 29 wieder so ausgebildet ist, dass sie den gesamten Querschnitt der Kammer ausfüllt.

Wird der Kolben 20 nach vorne in den Spritzenkörper 10 hineingedrückt, so bewegt sich auch diese Kolbenfläche 29 nach vorne und verringert das Volumen der ersten Kammer 46. Durch Erhöhung des Drucks reißt die Trennwand 47 und die flüssige Arzneimittelkomponente 63a wird in die vordere, kanülenseitige Kammer 48 verdrängt, wo sie sich mit der zweiten granulat-artigen Arzneimittelkomponente 63, vorliegend Mikro- und/oder Nanopartikel, unter Bildung einer Dispersion vermischt, wodurch das fertige Arzneimittel 63 entsteht. Dieser Zustand ist in Figur 5 gezeigt. Der Applikator befindet sich hier also in einer Art vorpräpariertem Zustand unmittelbar vor der Anwendung am Patienten.

Beispielsweise könnte der Behandler dann die Kanüle 50 in die stirnseitige zentrale Bohrung des Gehäuses 11 des Spritzenkörpers 10 einstecken und dabei gleichzeitig die vordere Folie 49, d. h. das Verschlusselement 49, der Depoteinheit 45 durchstoßen, sodass im Anschluss durch weiteres Verschieben der Kolbenfläche 29 durch ein weiteres hineindrücken des Kolbens 20 in den Spritzenkörper 10 das fertige Arzneimittel 62 in die Kanüle 50 gedrückt werden kann. Dieser Zustand des Applikators ist in Figur 6 gezeigt.

Es sei an dieser Stelle unter Verweis auf einen Vergleich der Ausführungsbeispiele gemäß den Figuren 1 und 2 und dem Ausführungsbeispiel gemäß den Figuren 4 bis 6 darauf hingewiesen, dass je nach Anwendungsart und Position der Spitze der Kanüle 50 eine Injektion am Glaskörper vorgenommen werden soll, der Facettenschliff an dem distalen Ende 52 der Kanüle 50 angeordnet sein kann. So befindet er sich bei den Ausführungsbeispielen gemäß den Figuren 1 bis 3 an der vom Spritzenkörper wegweisenden Seite der Kanüle 50 und bei dem Ausführungsbeispiel gemäß den Figuren 4 bis 7 auf der gegenüberliegenden Seite, d. h. die abgeschrägte Fläche weist schräg entgegen der Richtung, in die sich der Spritzenkörper 10 erstreckt.

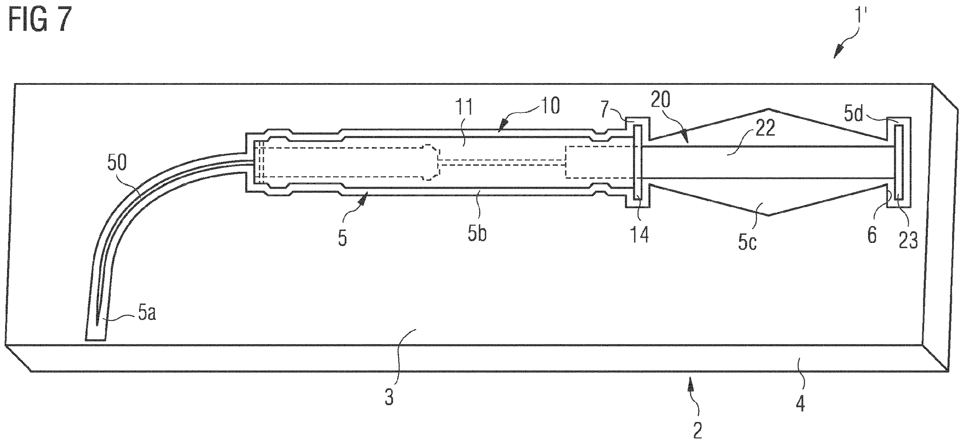

Figur 7 zeigt schließlich einen Applikator 1' mit zugehöriger Umverpackung 2. Der Spritzenkörper 10 mit dem Kolben 20 und einer darin befindlichen Depoteinheit 40 sowie mit der bereits anmontierten Kanüle 50 ist hierbei entsprechend dem Ausführungsbeispiel in Figur 1 ausgestaltet, nur dass vorliegend keine Abstützhilfe 17 vorhanden ist.

Die Umverpackung 2 besteht hier aus einer Art Grundgehäuse 4 mit einem darin aufgebrachten Aufnahmeraum 5 in Form einer Aufnahmemulde 5, deren Innenkonturen in einigen Teilbereichen 5a, 5b, 5c, 5d des Aufnahmeraums 5 an die Außenkonturen des Spritzengehäuses 10 und der Kanüle 50 angepasst sind. So befindet sich für die Kanüle 50 im vorderen Bereich ein erster entsprechend bogenförmiger schmaler Teilbereich 5a der Aufnahmemulde 5 für die Kanüle 50. An diesen schließt sich ein weiterer Teilbereich 5b an, dessen Konturen an die Form des Spritzenkörpers 10 angepasst sind. Auf der von der Kanüle 50 wegweisenden Seite des Spritzenkörpers 10 befindet sich anschließend an diesen zweiten Teilbereich 5b der Aufnahmemulde 5 ein dritter Teilbereich 5c der Aufnahmemulde 5 an, in welchem sich die Kolbenstange 22 erstreckt. In diesem Teilbereich 5c ist jedoch die Aufnahmemulde ein Stück breiter als die Kolbenstange 22 ausgestaltet, sodass ein Anwender die Kolbenstange 22 greifen kann, um das Spritzengehäuse 10 aus der Aufnahmemulde 5 herauszunehmen. Um den Kolben 20 exakt in seiner Position zu fixieren, befindet sich im hintersten Bereich der Aufnahmemulde 5 ein letzter, vierter Aufnahmebereich 5d für die Andruckplatte 23 am Ende der Kolbenstange 22, welcher wiederum an die Konturen der Andruckplatte 22 angepasst ist. Zwischen dem zweiten Teilbereich 5b und dem dritten Teilbereich 5c der Aufnahmemulde 5 befindet sich eine ringförmige Ausbuchtung 7 für den zum Kolben 20 weisenden endseitigen Kragen 14 des Gehäuses 11 des Spritzenkörpers 10 und entsprechend ist der dritte Aufnahmebereich 5c der Aufnahmemulde 5 durch eine ringförmige Einschnürung 6 von dem vierten Aufnahmebereich 5d für die Andruckplatte 23 getrennt, so dass die Andruckplatte 23 immer einen konstanten Abstand zum Kragen 14 einhält und der Kolben 20 nicht unbeabsichtigt in das Gehäuse 11 des Spritzenkörpers hineinbewegt werden kann.

Die Verpackung des Spritzenkörpers 10 mit der fertig montierten Kanüle 50 in diesen Grundkörper 4 mit dem Aufnahmeraum 5 geschieht vorzugsweise unter sterilen Bedingungen und innerhalb des sterilen Bereiches wird das Gehäuse 4 obenseitig mit einer Blisterfolie 3 abgedeckt und dicht verschlossen. Das Gehäuse 4 kann beispielsweise in einem Spritzgussverfahren oder in einem Tiefziehverfahren aus entsprechend starker Kunststofffolie hergestellt sein. Vorzugsweise wird darauf geachtet, dass das gesamte Gehäuse mit der Aufnahmemulde 5 so stabile Wände aufweist, dass es auch unter Krafteinwirkung von außen weitgehend formstabil bleibt. In diesem Fall kann beispielsweise auf eine zusätzliche Schutzkappe auf dem distalen Ende 52 der Kanüle 50 verzichtet werden.

Das Implantat kann beispielsweise aus Poly(D,L-Lactid-co-Glycolid) 50 : 50 bestehen und 700 Mikrogramm eines Cortisonderivats, wie beispielsweise Dexamethason, enthalten. Der Durchmesser eines solchen Implantats liegt beispielsweise bei etwa 0.5 mm ; die Länge beträgt etwa 6 mm. Solche Implantate können beispielsweise über eine Schmelzextrusion einer homogenen vorab hergestellten Pulvermischung hergestellt.

Es wird abschließend noch einmal darauf hingewiesen, dass es sich bei den vorhergehend detailliert beschriebenen Vorrichtungen lediglich um Ausführungsbeispiele handelt, welche vom Fachmann in verschiedenster Weise modifiziert werden können, ohne den Bereich der Erfindung zu verlassen. So könnte beispielsweise auch ein vorderer Bereich des Spritzenkörpers gebogen sein und/oder die Kanüle könnte verschiedene Krümmungswinkel aufweisen. Weiterhin schließt die Verwendung der unbestimmten Artikel "ein" bzw. "eine" nicht aus, dass die betreffenden Merkmale auch mehrfach vorhanden sein können.

### Bezugszeichenliste

1, 1' Applikator
2 Umverpackung
4 Grundgehäuse der Umverpackung
5 Aufnahmeraum / Aufnahmemulde
5a, 5b, 5c, 5d Teilbereiche
6 ringförmige Einschnürung
7 ringförmige Ausbuchtung
10 Spritzenkörper
11 Gehäuse
11b vorderes Gehäuseteil
11a hinteres Gehäuseteil
13 Hohlraum / Kolbenraum
14 Kragen
15 Griffmulden
16 stirnseitige Öffnung / Endbohrung
17 Abstützhilfe
20 Kolben
21 Stirnseite
22 Kolbenstange
23 Andruckplatte
27 Kolbenfläche
29 Kolbenfläche
30 Mandrin
31 Mandrin
40 Depoteinheit
41 Hohlraum
42 Führungsbohrung
43 Depoteinheit
44 Verschlusselement
45 Depoteinheit
46 hintere (erste) Kammer
47 Trennwand
48 vordere (zweite) Kammer
49 Verschlusselement / Folie
50 Kanüle
51 Kanülenende
52 distales (Kanülen-)Ende
53 schräge Fläche
54 Spitze
55 Spritzenkappe
56 Stopfen / Verschlusselement
60 Arzneimittel / Implantat
61 Arzneimittel / Implantat
62 Arzneimittel
63 Zwei-Komponenten-Arzneimittel
63a erste Arzneimittelkomponente
63b zweite Arzneimittelkomponente
dᵢ Innendurchmesser
dₐ Außendurchmesser
V Längsachse
T Tangente
r Krümmungsradius
α Winkel
β Winkel

## Patentansprüche

1. Applikator (1, 1'), insbesondere zur intravitrealen Applikation, umfassend
- einen Spritzenkörper (10) mit einem in einem Gehäuseteil (11a) beweglich gelagerten Kolben (20, 27, 29),
- eine Kanüle (2),
wobei zumindest ein Abschnitt der Kanüle (50) und/oder des Spritzenkörpers (10) gekrümmt ausgebildet sind.

2. Applikator nach Anspruch 1, wobei ein Winkel (α) einer Abweichung einer an einem distalen Ende (52) der Kanüle (50) anliegenden Tangente (T) von einer Richtung einer Längsachse (V) des Kolbens (20, 27, 29) mindestens 2°, vorzugsweise mindestens 10° und/oder maximal 80°, vorzugsweise maximal 90°, beträgt.

3. Applikator nach Anspruch 1 oder 2, wobei die Kanüle (50) einen über deren Länge gleichbleibenden Durchmesser (d,), vorzugsweise einen gleichbleibenden Innendurchmesser (di), aufweist,
und/oder
wobei die Kanüle (50) einen Innendurchmesser (di) von mindesten 0.5 mm aufweist, und/oder
wobei die Kanüle (50) einen Außendurchmesser (da) von höchstens 2.5 mm aufweist.

4. Applikator nach einem der vorhergehenden Ansprüche, wobei die Kanüle (50) an einem distalen Ende (52) spitz zuläuft.

5. Applikator nach einem der vorhergehenden Ansprüche, wobei eine Nettolänge der Kanüle (50) mindestens 1 cm und/oder höchstens 10 cm beträgt.

6. Applikator nach einem der vorhergehenden Ansprüche mit zumindest einem Arzneimittel (60, 61, 62, 63), vorzugsweise mit einer Depoteinheit (40, 43, 45) umfassend das zumindest eine Arzneimittel (60, 62, 63).

7. Applikator nach einem der vorhergehenden Ansprüche, umfassend einen, vorzugsweise zumindest abschnittsweise flexibel ausgebildeten, Mandrin (30, 31).

8. Applikator nach einem der vorhergehenden Ansprüche, wobei die Kanüle (50) und/oder der Spritzenkörper (10) und/oder die Depoteinheit (43, 45) ein Verschlusselement (44, 49) aufweisen.

9. Applikator nach einem der vorhergehenden Ansprüche, wobei der Spritzenkörper (10) zumindest eine Griffhilfe (15) und/oder eine Abstützhilfe (17) aufweist.

10. Applikator nach einem der vorhergehenden Ansprüche, umfassend eine Umverpackung (2), wobei die Umverpackung (2) einen formstabilen Aufnahmeraum (5) für den Spritzenkörper (10) und/oder die Kanüle (50) umfasst.

11. Applikator nach einem der vorhergehenden Ansprüche, wobei das Arzneimittel (60, 61, 62, 63) in flüssiger und/oder halbfester und/oder fester Form vorliegt.

12. Applikator nach einem der vorhergehenden Ansprüche, wobei das Arzneimittel (60, 61) ein Implantat (60, 61), insbesondere ein bioabbaubares Implantat (60, 61), umfasst.

13. Applikator nach einem der vorhergehenden Ansprüche,
wobei das Arzneimittel einen Wirkstoff umfasst, welcher ausgewählt ist aus
- einem Glucokortikoid, bevorzugt aus Dexamethason, Prednisolon oder Hydrocortison,
und/oder
- einem VEGF-Inhibitor, bevorzugt aus Ranibizumab, Bevacizumab und/oder Aflibercept,
und/oder
- einem Wirkstoff aus der Gruppe der Antiinfektiva, wie beispielsweise Aciclovir, Chloramphenicol, Fluorochinolonacetonid und/oder Gentamicin,
wobei das Arzneimittel besonders bevorzugt einen Wirkstoff umfasst, welcher ausgewählt ist aus Dexamethason, Ranibizumab, Bevacizumab, Aflibercept, Methotrexat, Vincristin, Carboplatin und/oder Hydrocortison, insbesondere aus Dexamethason, Ranibizumab, Bevacizumab oder Aflibercept.

14. Applikator nach einem der vorhergehenden Ansprüche zur Verabreichung von flüssigen, halbfesten und festen Arzneimitteln, bevorzugt von Liposomen, Mikropartikeln, Suspensionen, Lösungen, Gelen und/oder Implantaten in den Glaskörper, insbesondere von Implantaten.

15. Applikator gemäß einem der vorhergehenden Ansprüche zur Verwendung in der Augenheilkunde, bevorzugt zur Therapie von krankhaften Veränderungen des Auges, besonders bevorzugt zur Behandlung der altersbedingten Makuladegeneration, der Retinopathie, des Grauen Stars (Cataract), des Grünen Stars, der Hornhautentzündung (Keratitis), Color Vision Defect, Glaucoma, Herpes simplex-Infection, Endophthalmitis, Chorioditis, Iritis, Makulaödem, Uveitis, Entzündungen des posterioren Augensegments, Cytomegalie-Virus-Retinitis, Neovascularisation, bakterielle und virale Infektionen, Augentumorerkrankungen und/oder zur Immunsuppresion und/oder - stimulation, insbesondere zur Behandlung des diabetischen Makulaödems (DMÖ), des Makulaödems als Folge eines retinalen Venenverschlusses und von Entzündungen des posterioren Augensegments.
